# EUROPÄISCHE PATENTSCHRIFT

(11) **EP 2 096 982 B1**
(45) Veröffentlichungstag und Bekanntmachung des Hinweises auf die Patenterteilung: **13.04.2016**
(21) Anmeldenummer: 07846809.7
(22) Anmeldetag: 24.11.2007
(51) Int. Cl.: A61B 1/00, A61B 1/313, A61B 17/16, A61B 17/17, A61B 17/00

(54) **VORRICHTUNG UND VERFAHREN ZUM MINIMAL-INVASIVEN EINGRIFF AN DER WIRBELSÄULE**
APPARATUS AND METHOD FOR MINIMALLY INVASIVE INTERVENTION ON THE SPINAL COLUMN
DISPOSITIF ET PROCÉDÉ POUR INTERVENTION MINIMALE INVASIVE SUR LA COLONNE VERTÉBRALE

(30) Priorität: 27.11.2006 ES 200603026
(43) Veröffentlichungstag der Anmeldung: 09.09.2009
(73) Patentinhaber: Joimax GmbH, 76227 Karlsruhe (DE)
(72) Erfinder: MORGENSTERN LOPEZ, Rudolf, 08950 Esplugues de Llobregat (ES); RIES, Wolfgang, 76351 Linkenheim (DE)
(74) Vertreter: Lichti - Patentanwälte Partnerschaft mbB
(86) Internationale Anmeldenummer: PCT/EP2007/010238
(87) Internationale Veröffentlichungsnummer: WO 2008/064842

(56) Entgegenhaltungen:
- EP-A- 0 585 826
- EP-A- 1 468 652
- WO-A-00/76409
- WO-A-2006/044727
- WO-A-2006/091622
- DE-C1- 19 547 246
- US-A- 4 625 713
- US-A- 5 195 541
- US-A- 5 674 184
- US-A- 5 797 944
- US-A- 5 961 522
- US-A1- 2002 016 592
- US-A1- 2003 055 316
- US-A1- 2006 206 118

## Beschreibung

Die Erfindung betrifft eine Vorrichtung zum minimal-invasiven endoskopischen Eingriff an der Wirbelsäule. Die Erfindung betrifft insbesondere eine Vorrichtung zum Entfernen von Gewebe bei endoskopischen Eingriffen, vor allem zur Entfernung von Knochengewebe oder verbindendem Gewebe sowie anderen Gewebearten.

Die Erfindung beinhaltet insbesondere die Behandlung von Spinalstenosen, aber auch die vorbereitende Erweiterung von Zugangskanälen im Hinblick auf den endoskopischen Eingriff zur Behandlung von Bandscheibenvorfällen, wobei die Erfindung sich nicht auf diese Verwendungszwecke beschränkt.

Zur Behandlung eines Bandscheibenvorfalls der Wirbelsäule sind verschiedene endoskopische Techniken und Vorrichtungen bekannt. Im Kern wird beispielsweise, nachdem ein Schnitt in der Haut des Patienten erfolgt ist, zunächst (perkutan) ein längliches Element mit einer kegelförmigen abgerundeten Spitze eingeführt, dessen Zweck die Adaption des weichen Gewebes bis zum Umfeld der beschädigten, zu reparierenden Bandscheibe ist. Nachdem dieses erste längliche Element eingeführt wurde, wird über dieses eine feine Kanüle eingeführt, deren innerer Durchmesser mit dem äußeren Durchmesser des ersten länglichen Elements übereinstimmt. Der äußere Durchmesser der Kanüle kann zwischen 2 mm und 10 mm liegen, wobei am häufigsten Kanülen mit einem Durchmesser von etwa 6 mm benutzt werden. Diese Kanüle weist einen runden Querschnitt auf und kann unterschiedliche Formausbildungen an ihrem distalen Ende haben, wobei das distale Ende in der Regel eine abgeschrägte Form bezüglich der Achse der Kanüle aufweist, um so eine bessere Sicht auf den Arbeitsbereich zu ermöglichen. Nach Einführen der Kanüle wird das erste längliche Element entfernt, so dass ein offener Zugangskanal zur beschädigten Bandscheibe verbleibt, indem eine optische Sonde (Endoskop) eingeführt wird, die an diesen Kanal angepasst ist und die wiederum Kanäle für Druckwasser zur Säuberung und zum Absaugen von Material sowie Arbeitskanäle für Arbeitsinstrumente, wie Zangen oder dergleichen, zur Behandlung und zur Bearbeitung des (Bandscheiben-) Gewebes hat.

Allerdings kann sich das Problem ergeben, dass im Arbeitsbereich Knochengewebe oder Knochenwucherungen vorhanden sind, die die Kanüle hindern, bis in den Bereich der zu behandelnden Bandscheibe vorzudringen bzw. sich störend auf die Ausrichtung der Kanüle im Hinblick auf den Arbeitsbereich auswirken. Häufig ist es daher notwendig, ein schneidendes Werkzeug einzusetzen, um Knochengewebe abzuschneiden bzw. abzufeilen, um so Zugang zur zu behandelnden Stelle erhalten.

Hierzu bieten die derzeit bekannten Techniken verschiedene Lösungen. Bei einer ersten handelt es sich um ein Schneidwerkzeug von einer Größe, die es ermöglicht, durch einen Arbeitskanal der optischen Sonde eingeführt zu werden. Diese Lösung ermöglicht dem Nutzer Knochengewebe zu entfernen und dabei die optische Kontrolle seines Vorgehens zu bewahren. Allerdings besteht hier das Problem, dass das Werkzeug notwendigerweise einen sehr reduzierten Durchmesser haben muss (Höchstdurchmesser 3,5 mm) und der Vorgang zur Entfernung von Knochengewebe zu viel Zeit in Anspruch nehmen kann, was ein Nachteil für den Patienten ist. Eine zweite bekannte Lösung ist die Entfernung der optischen Sonde und die Nutzung eines Schneidwerkzeugs mit größerem Durchmesser. Hier besteht die Schwierigkeit darin, dass der Nutzer den Eingriff vornehmen muss, ohne unmittelbare Sicht auf das vorhandene weiche Gewebe zu haben, mit der sich daraus ergebenden Gefahr, Nervengewebe in spiralen Bereichen zu verletzen.

In beiden Fällen handelt es sich bei dem Schneidwerkzeug normalerweise um einen Zylinder, dessen distales Ende senkrecht zur Achse des Zylinders steht. Dieses distale Ende hat eine normalerweise sägeförmige Schneidenausbildung. Vor allem der Durchmesser des Werkzeugs ist für jede der beiden genannten Lösungen unterschiedlich.

Aus der WO 00/76409 A1 ist eine Vorrichtung zur Behandlung eines Bandscheibenvorfalls bekannt, wobei die Vorrichtung ein Endoskopiewerkzeug zum Entfernen von Weichteilen und eine Arbeitskanüle aufweist, durch welche das Werkzeug verschiebbar ist. Die Vorrichtung weist einen Werkzeugsatz auf, welcher aus Trokar-Röhren mit progressiv zunehmenden Außendurchmesser und hohlen Sägewerkzeugen gebildet ist.

Die Sägewerkzeuge sind auf die zugehörigen Trokar-Röhren aufsetzbar und auf der Röhre rotierbar, um das Knochenmaterial um das Facettengelenk der Wirbelsäule zu durchbohren.

Der Erfindung liegt die Aufgabe zugrunde, eine Vorrichtung und ein Verfahren vorzuschlagen, die unter Vermeidung der vorgenannten Nachteile insbesondere die Entfernung von Knochengewebe bei endoskopischen Eingriffen an der Wirbelsäule in effektiver Form ermöglichen und jederzeit die optische Kontrolle des Eingriffs durch den Benutzer erlauben.

Erfindungsgemäß wird die genannte Aufgabe mit einer Vorrichtung der eingangs genannten Art gelöst, welche die kennzeichnenden Merkmale des Anspruchs 1 aufweist.

Das Schneidwerkzeug der erfindungsgemäßen Vorrichtung kann von Hand (Bewegung der Schneide direkt durch die Hand des Benutzers), automatisch oder durch eine Kombination beider Vorgehensweisen bewegt werden. Für die beiden letzten Fälle verfügt die Vorrichtung vorzugsweise über eine Einrichtung für einen automatischen Antrieb, um dem mit der Schneide versehenen kanülenartigen Schneidwerkzeug eine sich wiederholende, schwingende Bewegung zu ermöglichen. Der Antrieb kann dabei in einem Handgriff angeordnet sein, wobei das Schneidwerkzeug bzw. die entsprechende Kanüle mit der sich relativ zum Handgriff bewegenden Abtriebsachse des Antriebs verbunden ist. Bei einer solchen schwingenden Bewegung kann es sich um eine Hin- und Herbewegung in Längsrichtung des Schneidwerkzeugs und/oder um eine hin- und hergehende Schwenkbewegung um die Achse des Schneidwerkzeugs handeln, vorzugsweise bis insgesamt 30°, höchst vorzugsweise weniger als 12°, das heißt entsprechend um 15° bzw. bis zu 6° in Bezug auf eine mittlere Gleichgewichtsposition. Die Schwingungen tragen dazu bei, dass der Knochen zuverlässig und bequem geschnitten wird. Die Einrichtungen zur Erzeugung der Bewegungen können in bekannter Art ausgestaltet sein, einschließlich Motoren, elektromagnetischen Mechanismen usw.

Wie sich aus Vorstehendem ergibt, beinhaltet die Erfindung auch ein Schneidwerkzeug zur Beseitigung von Gewebe bei endoskopischen Eingriffen, insbesondere einsetzbar als Schneidwerkzeug der erfindungsgemäßen Gesamtvorrichtung. Das Werkzeug beinhaltet ein Element in Form einer hohlen Kanüle, an deren distalem Ende sich eine Schneide befindet und ist dadurch charakterisiert, dass die Öffnung des genannten distalen Endes in Bezug auf eine symmetrische Achse des kanülenartigen Elements eine abgeschrägte Form hat. Weil die erwähnte Schneide sich am entferntesten distalen Bereich an der Stirnseite der Wand des kanülenförmigen Elements befindet, ist der innere Hohlraum desselben frei, um eine optische Sonde aufzunehmen.

Damit das Schneidwerkzeug beim Einführen kein Gewebe verletzt, sollte es vorzugsweise ein Schnittprofil mit einer kegelförmigen Oberfläche aufweisen, die die Klinge mit der Außenseite der Wand der Kanüle verbindet. Vorzugsweise sollte die Klinge mindestens auf einer radialen Entfernung zur Innenseite der Kanülenwand liegen, die lediglich ein Viertel des radialen Abstandes zur Außenseite beträgt. Vorzuziehen ist, dass die Schneide mit dem distalen Ende der inneren Seite der Kanülenwand übereinstimmt bzw. fluchtet. In bevorzugter Ausgestaltung ist die Schneide sägeförmig ausgebildet.

Durch die Form der Schneide kann das Schneidwerkzeug in ähnlicher Weise, wie bereits bekannte Kanülen in den menschlichen Körper eingeführt werden, ohne dass dadurch Schnittverletzungen am Gewebe im Einführungsbereich verursacht werden. Außerdem ermöglicht die genannte Kegelform eine korrekte Sicht auf den zu schneidenden Bereich aus dem Inneren heraus mittels eines Endoskops.

Während das vorstehend beschriebene Werkzeug mit abgeschrägtem distalen Ende und einer Schneidkante lediglich am weitesten vorstehenden Bereich, insbesondere zur Abtrennung von Knochenwucherungen im Einführungsbereich der Werkzeuge zum Wirbelkanal zum Einsatz kommt, sieht die Erfindung in einer weiteren Ausgestaltung zum Bearbeiten zentralerer Verengungen zusätzlich einen durch das Endoskop hindurchschiebbaren Meißelfräser vor, der insbesondere hohlzylindrisch ausgebildet ist und an seinem stirnseitigen Ende eine kreissymmetrische Zähnung aufweist.

Da mit dem Arbeiten mit einem solchen Fräser eine Führung desselben gegeben ist und damit die Gefahr von Beschädigungen nicht zu beschädigenden Gewebes oder auch Nerven reduziert bzw. ausgeschaltet wird, sieht die Erfindung in einer äußerst bevorzugten Ausgestaltung eine Vorrichtung vor, die mindestens ein durch das Endoskop durchschiebbares Verankerungswerkzeug aufweist, wobei die Vorrichtung auch zwei oder mehrere derartige, gegebenenfalls alternativ einsetzbare Verankerungswerkzeuge aufweisen kann. Die Verankerungswerkzeuge sind an ihrem distalen Ende derart ausgebildet, dass sie insbesondere am hinteren Längsband der Wirbelsäule festlegbar sind. Zur Handhabung ist vorgesehen, dass das Verankerungswerkzeug an seinem rückwärtigen (proximalen) Ende mit einer Verbindungsausbildung zur drehfesten Verbindung mit einem Griff oder dergleichen versehen ist, wobei insbesondere das Verankerungswerkzeug in seinem rückwärtigen Bereich distal zur Verbindungsausbildung mit Graduierungen, insbesondere in Form von sich um den Teilumfang des Verbindungswerkzeugs senkrecht zu dessen Längsachse erstreckenden Einschnitten versehen ist.

Eine bevorzugte Ausgestaltung sieht vor, dass das Verankerungswerkzeug eine Endoahle ist, wobei die Endoahle vorzugsweise eine scharfe distale Spitze aufweist. Eine andere Ausgestaltung sieht vor, dass das Verankerungswerkzeug ein Endospatel ist, wobei der Endospatel vorzugsweise an seinem distalen Ende mit einer stirnseitigen Schneide versehen ist. Schließlich ist eine äußerst bevorzugte Ausgestaltung der Erfindung dadurch gekennzeichnet, dass das Verankerungswerkzeug ein Endoelevator ist, wobei insbesondere der Endoelevator vorzugsweise in seinem distalen Endbereich zunächst eine Verjüngung und an seinem äußersten distalen Ende wiederum eine Verdickung aufweist.

Das Arbeiten mit der erfindungsgemäßen Vorrichtung gestaltet sich folgendermaßen: Das Schneidwerkzeug wird um einen begrenzten Winkelbereich verschwenkt, wobei es alternativ oder zusätzlich zyklisch axial, also stoßend, bewegt werden kann. Das Schneidwerkzeug kann über einen Winkelbereich bis zu 30°, insbesondere vorzugsweise weniger als 12° verschwenkt wird. Hinsichtlich der Behebung zentral nahen Stenosen wird durch den Arbeitshohlraum eines bis in den Wirbelsäulenbereich eingeführten Endoskops ein Verankerungswerkzeug eingeführt und im Bereich des hinteren Längsbandes verankert, wobei entweder als Verankerungswerkzeug eine Endoahle eingeführt und mit ihrer scharfen Spitze stoßend im Bereich am hinteren Längsband oder benachbarten Knochenbereichen axial verankert wird, als Arbeitswerkzeug ein Endospatel mit abgeflachtem distalen Ende eingeführt und zwischen hinterem Längsband und Knochen durch axiale Krafteinwirkung verankert wird oder aber als Arbeitswerkzeug ein Endoelevator mit einem mit einer Hinterschneidung versehenen Verdickung am distalen Ende eingeführt und zwischen Knochen und hinterem Längsband an letzterem auf Zug verankert wird.

Zum eigentlichen Bearbeiten der zentral nahen Stenose durch den Meißelfräser ist vorzugsweise vorgesehen, weiterhin über das Arbeitswerkzeug als Führungswerkzeug ein Meißelfräser eingeführt und mit diesem das zu entfernende Knochenmaterial durch zumindest eine Schwenkbewegung des Meißelfräsers entfernt, wobei der Meißelfräser ebenfalls gedreht und/oder axial zyklisch bewegt werden kann. Der Antrieb erfolgt vorzugsweise motorisch.

Weitere Vorteile und Merkmale der Erfindung ergeben sich aus den Ansprüchen und aus der nachfolgenden Beschreibung, in der Ausführungsbeispiele der Erfindung unter Bezugnahme auf die Zeichnung im Einzelnen erläutert sind. Dabei zeigt bzw. zeigen:
- Fig. 1: den unteren Teil einer Wirbelsäule zur Veranschaulichung der entsprechenden körperlichen Gegebenheiten;
- Fig. 2: in schematischer Darstellung eine beschädigte Bandscheibe (Bandscheibenvorfall), die Druck auf Nervenelemente verursacht sowie ein längliches Element mit runder kegelförmiger Spitze und ein erfindungsgemäßes Schneidwerkzeug in der Nähe des Bereichs, in dem operiert wird;
- Fig. 3.1: einen vergrößerten Vertikalschnitt durch den distalen Endbereich eines erfindungsgemäßen Schneidwerkzeugs;
- Fig. 3.2: eine achssenkrechte Seitenansicht auf den distalen Endbereich eines erfindungsgemäßen Schneidwerkzeugs;
- Fig. 3.3: eine vergrößerte perspektivische Darstellung des distalen Endbereichs des erfindungsgemäßen Schneidwerkzeugs;
- Fig. 3.4: eine Seitenansicht des distalen Endbereichs einer anderen Ausführungsform des erfindungsgemäßen Schneidwerkzeugs;
- Fig. 4: eine Seitenansicht eines erfindungsgemäßen Schneidwerkzeugs mit einem länglichen Element mit abgerundeter kegelförmiger Spitze (einer Sonde) im Inneren des Schneidwerkzeugs;
- Fig. 5.1: einen Längsschnitt durch das distale Ende eines in seinem Schneidbereich abgewandelten erfindungsgemäßen Schneidwerkzeugs;
- Fig. 5.2: eine Draufsicht auf das distale Ende des abgewandelten erfindungsgemäßen Schneidwerkzeugs gemäß Fig. 5.1;
- Fig. 6: eine schematische Darstellung einer erfindungsgemäßen Vorrichtung mit einem erfindungsgemäßen Schneidwerkzeug im Längsschnitt;
- Fig. 7: eine Seitenansicht einer weiteren Ausgestaltung einer erfindungsgemäßen Vorrichtung;
- Fig. 8 u. 9: schematische Darstellungen der zum Einsatz bereiten erfindungsgemäßen Vorrichtung;
- Fig. 10: eine Endoahle als Führungselement für einen Meißelfräser in perspektivischer Seitenansicht;
- Fig. 10.1: eine vergrößerte Darstellung der distalen Spitze der Endoahle der Fig. 10;
- Fig. 11: einen am distalen Ende geschärften Endospatel als Führung für einen Meißelfräser in perspektivischer Darstellung;
- Fig. 11.1: ein vergrößertes distales Ende des Endospatels der Fig. 11;
- Fig. 11.2: einen Längsschnitt durch das distale Ende eines Endospatels;
- Fig. 12: einen Endoelevator mit abgestumpftem distalen Ende als Führung für einen Meißelfräser in perspektivischer Darstellung;
- Fig. 12.1: eine vergrößerte Darstellung des distalen Endes des Endoelevators der Fig. 12;
- Fig. 12.2: einen vergrößerten Längsschnitt durch das distale Ende des Endoelevators;
- Fig. 13: eine Seitenansicht eines Meißelfräsers mit hohlem Schaft;
- Fig. 13.1: eine vergrößerte Seitenansicht des distalen Endes des Meißelfräsers der Fig. 13;
- Fig. 13.2: einen vergrößerten Längsschnitt durch das distale Ende des Meißelfräsers der Fig. 13;
- Fig. 14.1-14.3: Darstellung zum Zusammenwirken von Endoahle, Endospatel und Endoelevator mit einem Meißelfräser der Fig. 13; und
- Fig. 15: eine Seitenansicht eines Griffes zur Verbindung mit den vorgenannten Werkzeugen.

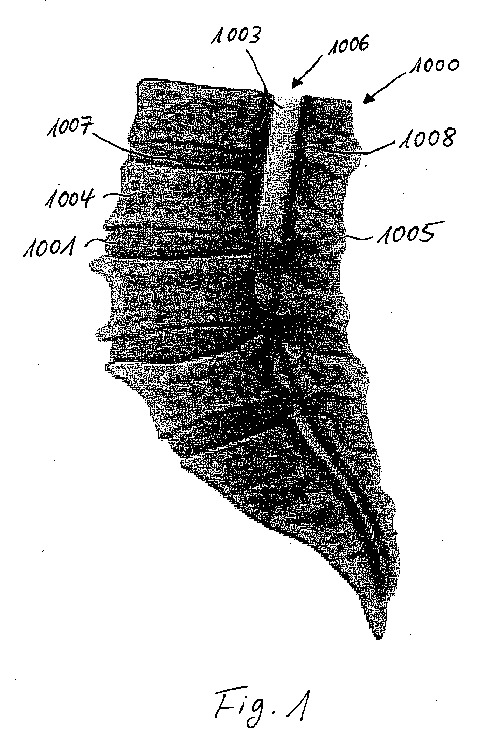

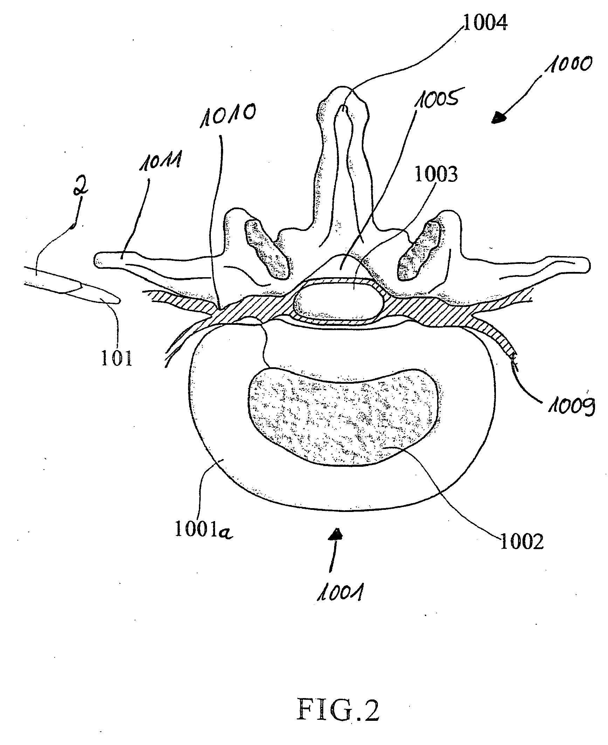

Die Fig. 1 zeigt im Längsschnitt den unteren Bereich einer Wirbelsäule 1000 mit Wirbeln (Vertebra) 1004 und von diesen nach hinten (dorsal) fortstehenden Dornfortsatz (Processus spinosus) 1005, zwischen denen sich - im dargestellten Schnitt - die den Wirbelkanal 1006 der Wirbelsäule 1000 bildenden Wirbellöcher (Foramen vertebrae) befinden. Zwischen den Wirbeln 1004 befinden sich die Zwischenwirbelscheiben oder Bandscheiben 1001 mit ihrem Kern 1002 (Fig. 2) und ihrem Ring (Anulus) 1001a.

Die Wirbel sind an der Vorderseite (ventral) des Wirbelkanals durch das vordere Längsband 1007 (Ligamentum longitudinale anterius) miteinander verbunden, während sich an der Rückseite des Wirbelkanals 1006, vor den Dornfortsätzen 1005, das hintere Längsband (Ligamentum longitudinale posterius) 1008 befindet, das mit den Wirbeln nur lose, aber fest mit den Bandscheiben 1001 verbunden ist. Durch den Wirbelkanal 1006 erstreckt sich Nervengewebe 1003, wobei einzelne Nerven 1009 (Fig. 2) seitlich zwischen den Wirbeln 1004 austreten. Seitlich des Wirbelkanals 1006 (durch das Nervengewebe 1003 verdeckt und daher in der Fig. 1 nicht erkennbar) findet sich jeweils ein sogenanntes gelbes Band (Ligamentum
flavum) als jeweils zwischen zwei Wirbeln gelegenes, die Wirbelsäule stabilisierendes Band.

Wie insbesondere aus der Fig. 2 ersichtlich ist, besteht insbesondere die Möglichkeit des minimal-invasiven Zugangs zum Wirbelkanal 1005 auf der Höhe der Bandscheiben 1001, beispielsweise zur Behebung von Bandscheibenvorfällen, die auf das Nervengewebe 1003 im Wirbelkanal 1005 drücken und zu Schmerzen führen.

Es ist eine Knochenwucherung 1010 eines Querfortsatzes (Processus transversus) 1011 des Wirbels 1004 in Richtung auf den Wirbelköper bzw. die Bandscheibe hin ersichtlich, die den Zugang zum Zentralbereich des Wirbelkanals 1005 einengt und verhindert, dass ein Hohlrohr mit hinreichend großem Durchmesser zum Einführen eines Endoskops eingeschoben werden kann.

Das erfindungsgemäße Schneidwerkzeug 2 ist an seinem distalen Ende zum Abtragen der Knochenwucherung 1010 ausgebildet.

Hierzu wurde schon bisher zunächst ein längliches Führungselement eingeführt, über welches, gegebenenfalls im Rahmen eines Dilatationsvorganges eine oder mehrere Kanülen, insbesondere eine Kanüle mit einem distalen Ende mit allgemein kegelförmiger Geometrie in Bezug zur Symmetrieachse eingeführt wurde, wodurch insbesondere das Mitreißen von sich am Operationsbereich befindlichem Gewebe hin zu dem behandelten Bereich verhindert wird. Beispielsweise kann das distale Ende eine flache Form haben, es sind aber auch andere Formen möglich und bekannt, sofern das Ende an einem ersten Teil distaler als an einem zweiten Teil ist, also eine allgemeine schräge Form in Bezug auf die Symmetrieachse der Kanüle aufweist.

Die Ränder, die durch die Wand der Kanüle definiert sind, sind abgerundet, um der Gefahr einer Gewebeverletzung während der Einführung der Kanüle entgegenzuwirken.

Durch den hohlen Bereich der Kanüle werden Arbeitsinstrumente bzw. eine optische Sonde bzw. ein Endoskop eingeführt. Im letzteren Falle, um Bilder des Arbeitsbereichs erhalten zu können. Zusätzlich können Eingangs- und Ausgangskanäle in der Kanüle zum Durchspülen mit Druckwasser vorhanden sein. Dieses Druckwasser wird genutzt, um Reste zu entfernen und ein sauberes Kamerabild des Operationsbereichs zu erhalten. Ein Endoskop ist ein Gerät mit einem im Wesentlichen zylindrischen Hauptkörper mit einem optischen Kanal, der gegebenenfalls einen Lichtleiter aufweist, und durch den aus dem distalen Ende des Geräts zur Beleuchtung des Umgebungsbereichs Licht austreten und von dort ein Bild eintreten kann, das am proximalen Ende über ein Mikroskop direkt oder einem Bildwandler und einem Bildschirm indirekt beobachtet werden kann. Im vorliegenden Fall des minimal-invasiven operativen Eingriffs weist der längliche Hauptkörper des Endoskops auf jeden Fall noch einen hohlen Arbeitskanal auf, durch den Arbeitsinstrumente vom proximalen Ende bis zum distalen Ende hindurch- und eingeführt werden können.

Die Fig. 2 zeigt die Einführung eines erfindungsgemäßen Schneidwerkzeugs oder einer Schneidkanüle, wie sie im Folgenden genauer beschrieben wird, über ein längliches Führungselement 101 von der Seite her zum Wirbelkanal 1006. Das längliche Element 101 wird durch einen Schnitt in der Haut des Patienten in dessen Körper eingeführt. Die kegelförmige runde Spitze dient dazu, körpereigenes Gewebe zur Seite zu drücken, um die Einführung einer Kanüle für das Endoskop zu ermöglichen, ohne dass diese Schäden im Körper verursacht. Die Kanüle hat einen Innendurchmesser, der ungefähr dem Außendurchmesser des länglichen Elements 101 entspricht. Auf diese Art wird ein Einschieben und Abtrennen von Gewebe in eine sonst mögliche Spalte zwischen dem länglichen Element 101 und der Kanüle vermieden. Nachdem die Kanüle eingeführt worden ist, wird das längliche Element 1001 entfernt, so dass der Innenbereich der Kanüle hohl ist. Der Innenbereich der Kanüle wird vom Benutzer als Arbeitsbereich benutzt und es können Schneidinstrumente, optische Sonden (Endoskope), Pinzetten usw. eingeführt werden. Des Weiteren können Arbeitskanäle im Inneren der Sonde vorhanden sein, um einen Fluss von unter Druck stehender wässriger Flüssigkeit zu schaffen, die zur Sauberhaltung des Endoskops dient, so dass die Sicht auf den Operationsbereich ermöglicht wird und zur Entfernung von Resten, die während des Eingriffs entstehen, genutzt werden kann. Alle Instrumente, die in die Kanüle eingeführt werden, haben eine längliche Form. So haben z.B. die bisher bekannten Schneidinstrumente eine längliche Zylinderform mit einem schrägen, sägeblattförmigen Ende, welches das Schneiden erleichtert.

Der Durchmesser der Kanüle ist aus offensichtlichen Gründen eingeschränkt (Beschränkung der Gewebeausdehnung und des durchzuführenden Schnitts). Als Folge dieser Einschränkung erlaubt das Innere der Kanüle lediglich die gleichzeitige Einführung von einer optischen Sonde mit einem zusätzlichen Arbeitskanal für ein Schneidwerkzeug mit einem sehr reduzierten Durchmesser, das wenig nützlich für die Anforderungen beim Schneiden von Knochengewebe sein kann. In diesen Fällen ist es notwendig, die optische Sonde aus dem Inneren der Kanüle zu entfernen und eine nicht-optische Sonde einzuführen, die über einen breiteren Arbeitskanal für ein Schneidwerkzeug von größerem Durchmesser verfügt. Das Schneiden des Knochengewebes erfolgt in diesem Falle blind, d.h. ohne die Möglichkeit der Sicht auf das weiche Gewebe, was offensichtlich sehr gefährlich ist, da Nervengewebe irreparabel beschädigt werden kann und der Erfolg des Eingriffs in der Fingerfertigkeit des Operateurs liegt.

Zur Vermeidung dieser Schwierigkeiten ist die Kanüle erfindungsgemäß als kanülenartförmiges Schneidwerkzeug 2 ausgebildet. Dieses hat ein distales Ende 22 mit abgeschrägter Form zur Symmetrieachse 25 der Kanüle. Die Größe des Schneidwerkzeugs 2 erlaubt die Einführung in den Körper in der zuvor beschriebenen Form und ermöglicht gleichzeitig eine optische Sonde durch ihren Innenbereich zu legen, in ähnlicher Form wie bei bereits bekannten Kanülen.

Das charakteristische Merkmal der vorliegenden Erfindung ist, dass das Schneidwerkzeug 2, das den Kerngegenstand der Erfindung bildet, über eine Schneide 26 am distalsten Bereich des distalen Endes, also am stirnseitigen Rand der Kanülenwand 21 verfügt.

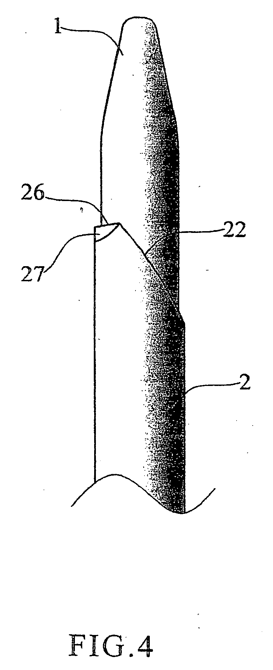

Der Umstand, dass sich die Schneide 26 am stirnseitigen Rand der Kanülenwand befindet, führt dazu, dass das Schneidwerkzeug 2, das den Gegenstand der Erfindung bildet, in ähnlicher Form, wie eine Kanüle der endoskopischen Systeme bekannter Art in den Körper eingeführt werden kann. Denn wenn ein längliches Element 101 eingeführt wird, das den gesamten Innenbereich ausfüllt, kann die Schneide 26 nicht das Gewebe trennen, wie aus Fig. 4 ersichtlich wird. Andererseits erlaubt die allgemeine schräge Form des distalen Endes des Schneidwerkzeugs 2 eine korrekte Sicht des zu schneidenden Bereichs vom Inneren des kanülenartigen Schneidwerkzeugs 2 aus.

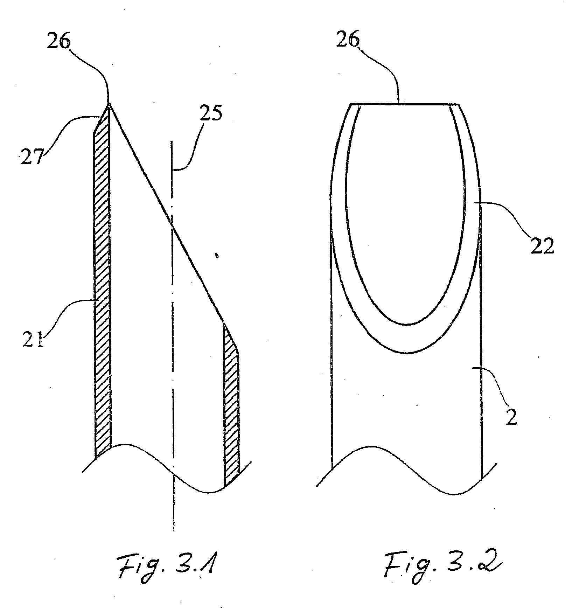

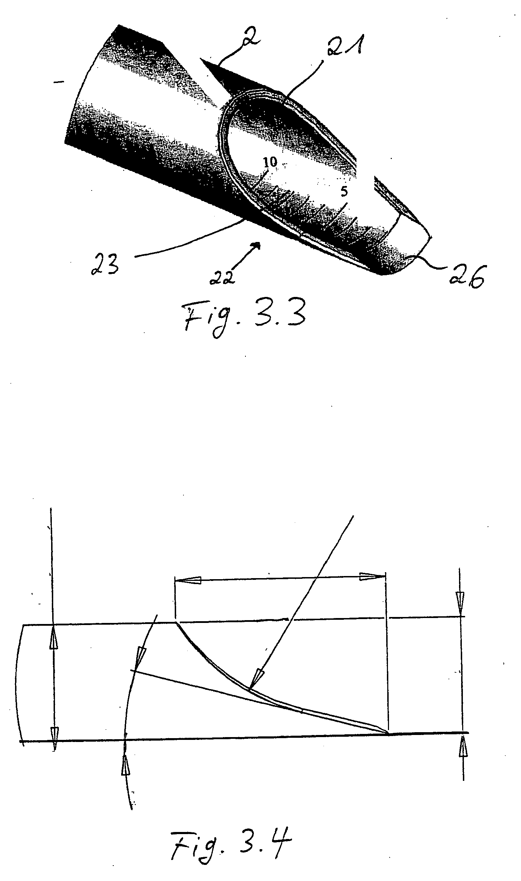

Im Falle des in Fig. 3 gezeigten Beispiels liegt die Schneide 26 auf der inneren Seite der Kanülenwand 21. Des Weiteren besteht die Schneide 26 aus einem Schnitt 27 in der Kanülenwand 21 und zwar schräg zu der Außenseite der Wand nach innen im Bereich des distalen Endes 22 des Werkzeugs 2 (Fig. 3, 4).

Die Fig. 3.3 zeigt, dass sich auf der Innenseite des Fortsatzes des Schneidwerkzeugs 2 eine Graduierung 23 oder Skala, beispielsweise durch eingeäzte transversale Vertiefungen, befindet. Weiterhin ist die Kante der stirnseitigen Öffnung des Schneidwerkzeugs 2 außer im Bereich an der vorderen Stirnseite befindlichen scharfen Kante 26 abgerundet. Schließlich ist aus Fig. 2 ersichtlich, dass bei der hier dargestellten Ausführungsform die Stirnseite des Schneidwerkzeugs 2 nicht lediglich abgeschrägt ist, sondern in Seitenansicht von der proximalen Seite der Öffnung her zunächst bogenförmig und erst in ihrem distalen Endbereich gestreckt schräg verläuft, wobei sie hierbei vorzugsweise zur Längsachse bzw. zur Seitenwandung einen Winkel in der Größenordnung von 10 bis 20° einschließt. Der Durchmesser eines erfindungsgemäßen kanülenartigen Schneidwerkzeugs 2 sollte größer als 3 mm sein und vorzugsweise im Bereich von 5 bis 7 mm liegen.

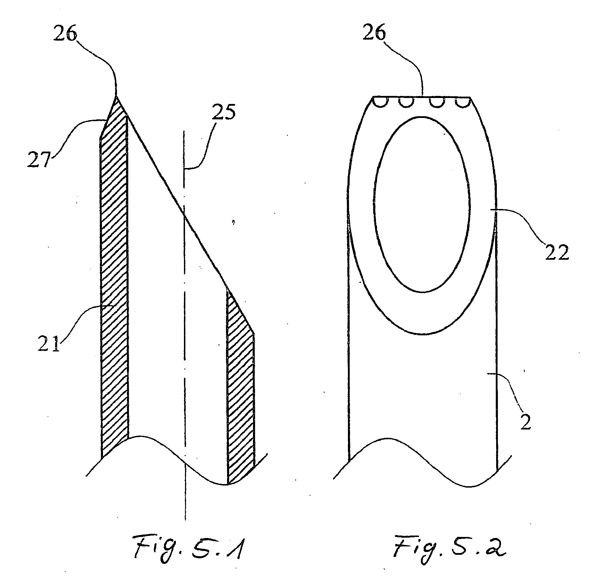

Die Fig. 5 zeigt eine Alternative der Ausgestaltung des Schneidwerkzeugs 2, das den Gegenstand dieser Erfindung bildet. In diesem Fall ist die Schneide 26 sägeförmig und deckt sich nicht mit der Innenseite der Kanülenwand 21. Allerdings verursacht auch dieses Werkzeug keine Schnittverletzungen am Gewebe, wenn es in den zuvor beschriebenen Weisen eingeführt wird.

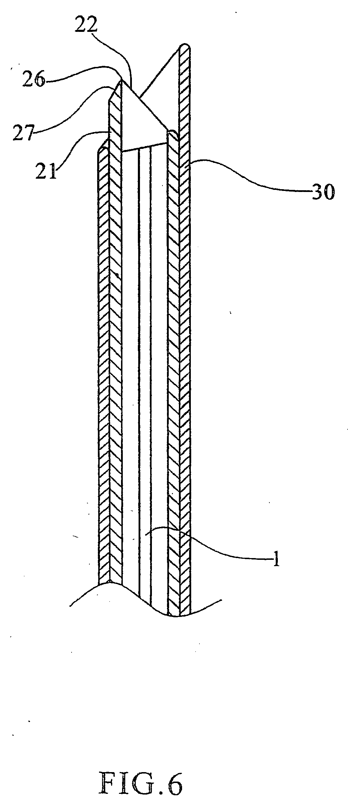

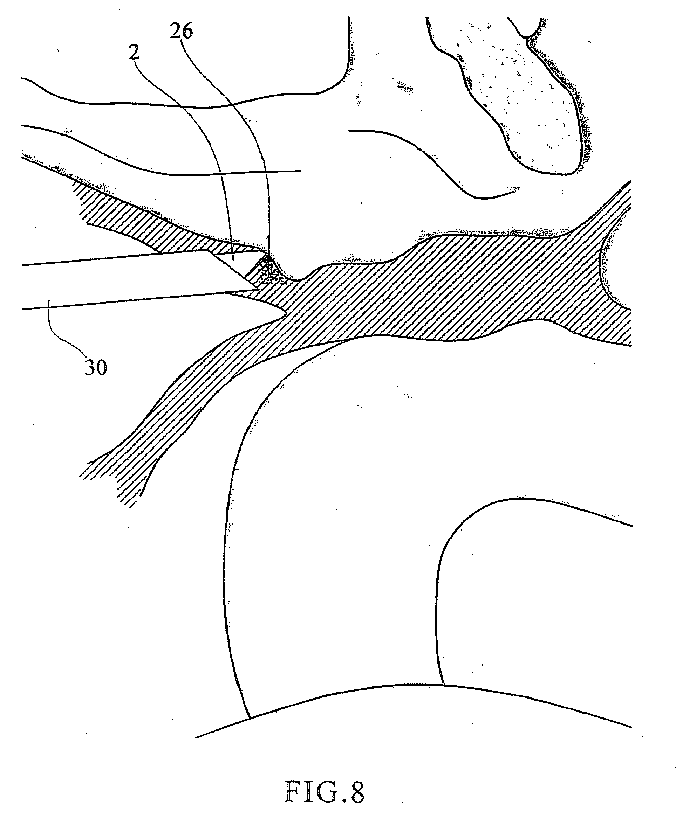

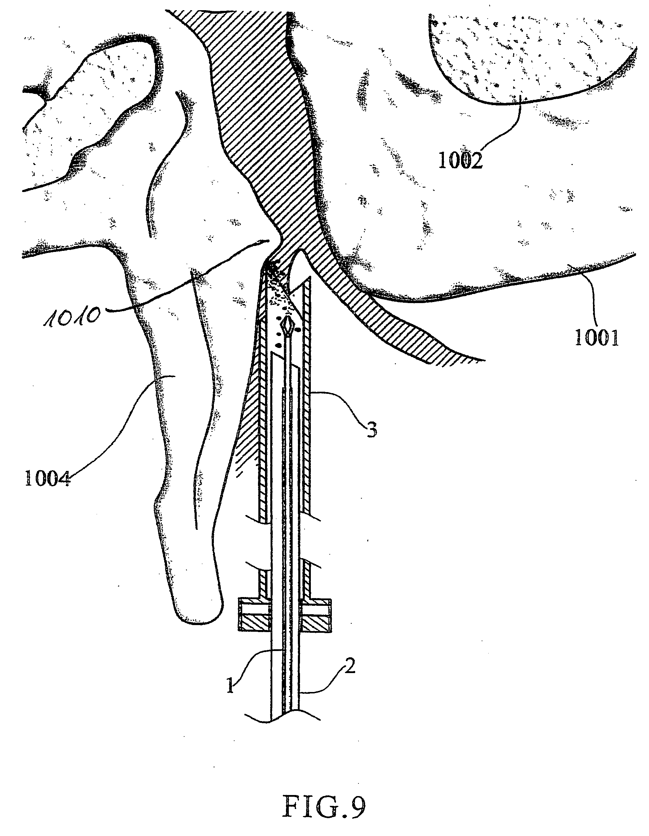

In den in den Fig. 3.1, 3.2 und 5.1, 5.2 dargestellten Beispielen wird die Öffnung des distalen Endes des Schneidwerkzeugs 2 durch eine Ebene definiert. Allerdings kann diese Öffnung auch andere Formen annehmen, wie z.B. eine gebogene Oberfläche (Fig. 3.3) usw. Die Schneidwerkzeuge 2 können einen ähnlichen Durchmesser, wie den der äußeren Kanülen der Vorrichtungen gemäß dem Stand der Technik haben, haben aber insbesondere und vorteilhafterweise einen etwas größeren Durchmesser, so dass auch Endoskope mit größerem Durchmesser als bisher eingesetzt werden können. Im Fall eines Systems gemäß der vorliegenden Erfindung vereint das Schneidwerkzeug 2 die Funktionen der Außenkanüle und die des Schneidwerkzeugs. Das System gemäß dieser Erfindung wird durch eine optische Sonde 1 (Endoskop) vervollständigt, die sich im Inneren des kanülenartigen Schneidwerkzeugs 2 mit der Schneide 26 befindet und die über einen Arbeitskanal für Werkzeuge verfügt. Es ist von Vorteil, eine weitere, äußere hohle Kanüle 30 mit einem Durchmesser, der größer oder gleich ist, als der des Schneidwerkzeugs 2 mit Schneide 26 ist, einzusetzen. Diese externe Kanüle 30 kann an das Schneidwerkzeug 2 mit Schneide 26 angepasst sein und hinsichtlich ihrer Ausgestaltung den bekannten äußeren Kanülen entsprechen (nicht ihres Durchmessers). Durch diese Kanüle 30 erhält man eine größere Kontrolle über den Arbeitsbereich, z.B. durch das Einflößen von Druckflüssigkeit durch einen Arbeitskanal. Die Fig. 6 zeigt eine Vorrichtung dieser Art. Die Fig. 8 und 9 zeigen schematische Darstellungen der Arbeitsweise. Die äußere Kanüle 30 ist unbeweglich, während das Schneidwerkzeug 2 sich bewegt, um die Knochenwucherung 1010 zu schneiden. Unter diesen Bedingungen kann z.B. Druckflüssigkeit durch den Arbeitskanal gespült werden, so dass die Dimension des Arbeitsraums, durch den die Druckflüssigkeit gespült wird, nicht mit der Bewegung des Schneidwerkzeugs 2 variieren.

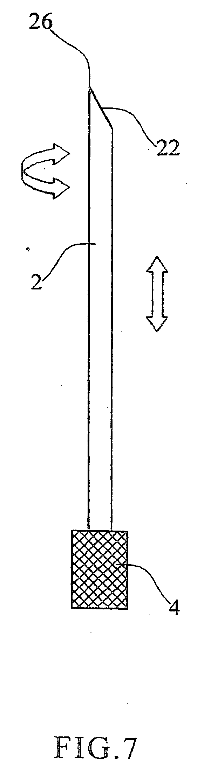

Die Erfindung hat also auch den Vorteil, schneiden zu können, ohne die Arbeitskanäle der optischen Sonde zu belegen. In der Fig. 9 wird eine endoskopische Pinzette durch einen Arbeitskanal der Sonde 1 gelegt. Des Weiteren kann, wie man aus den Fig. 7 bis 9 erkennt, das System über Mittel 4 verfügen, um eine alternative Bewegung des Schneidwerkzeugs 2 zu ermöglichen, um so das Schneiden des Knochengewebes zu vereinfachen. Es existieren verschiedene verfügbare Techniken, um diese Art der Bewegung zu ermöglichen: pneumatische, magnetische, elektrische, mechanische Systeme usw. Daher wird deren Beschreibung hier nicht weitergehend vertieft. Diese Mittel können Mittel enthalten, um eine Bewegung der Länge nach zu einer Achse des Schneidwerkzeugs 2 oder eine alternative Bewegung um eine Achse 25 des Werkzeugs herum, vorzugsweise mit einer Einschränkung der Schwenkbewegung auf einen Radius von bis zu 15°, insbesondere weniger als 6° in Bezug auf eine Gleichgewichtsposition zu ermöglichen, mit also einem Gesamtschwenkbereich von 30° bzw. vorzugsweise bis zu 12°.
Bezüglich der Größe kann das Schneidwerkzeug 2 einen inneren Durchmesser zwischen 2,7 mm und 7,3 mm haben, allerdings vorzugsweise zwischen 3,2 und 6,1 mm. Die Länge des Schneidwerkzeugs und der weiteren Mittel kann denen der bereits bekannten Systeme entsprechen.

Das Verfahren beinhaltet also bis hierin folgendes:
Bei einem Hautschnitt wird zunächst mindestens ein stabförmiges Werkzeug eingeführt. Vorzugsweise werden mehrere rohrförmige Dilatationswerkzeuge mit anwachsendem Durchmesser übereinander eingeführt, bis schließlich das erfindungsgemäße Schneidwerkzeug eingeführt werden kann. Die innerhalb desselben anliegenden Dilatationsstäbe werden herausgenommen und anschließend durch das Schneidwerkzeug ein Endoskop bis zum distalen Bereich des Schneidwerkzeugs eingeführt, so dass der Arbeitsbereich der Schneide des Schneidwerkzeugs überwacht werden kann.
Anschließend kann das Arbeiten mit dem Schneidwerkzeug durch rhythmisches oder zyklisches Stoßen und Hin- und Herschwenken erfolgen, um eine Knochenwucherung, ein Knochenvorsprung oder dergleichen zu entfernen.
Insbesondere, wenn sich die durch Knochenmark real bedingte Enge im Eingangsbereich des Einführungskanals findet, wie dies bei der Verengung 1010 der Fig. 1 der Fall ist, ist ein "freihändiges Arbeiten", wie vorstehend beschrieben, möglich. Dies ist aber weniger oder nicht mehr der Fall, wenn Knochenmaterial zentraler und näher beim Nervengewebe 1003 zu entfernen ist, da ein Abrutschen der scharfen Kante 26 des Schneidwerkzeugs 2 zu einer Beschädigung von Nerven führen kann. In diesem Fall ist zumindest eine gewisse sichere Führung von kanülenförmigen Arbeitswerkzeugen erforderlich. Es ist daher notwendig, Führungswerkzeuge für kanülenförmige Arbeitswerkzeuge sicher mit ihrem distalen Ende im Wirbelkanal bzw. in diesem begrenzenden Material zu verankern.

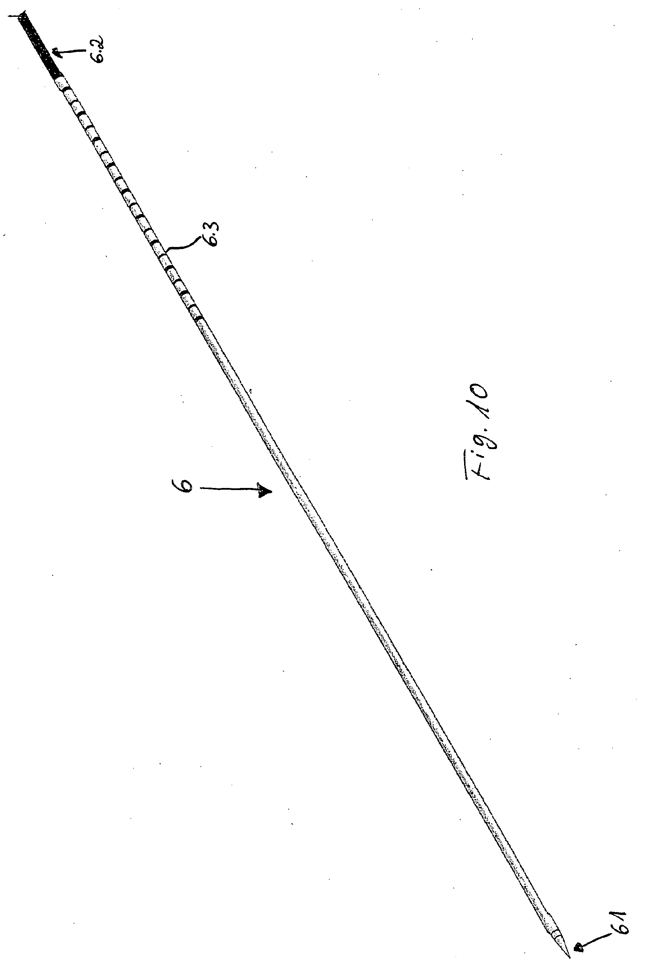

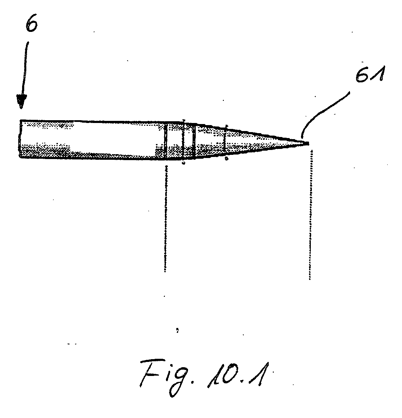

Hierzu sieht die Erfindung in einer ersten erfindungsgemäßen Ausgestaltung zunächst eine Endoahle 6 vor, wie sie in der Fig. 10 dargestellt ist. Bei der Endoahle 6 der Fig. 10 handelt es sich um einen massiven länglichen Stab mit einem spitzen, scharfen distalen Ende 6.1, einem rückwärtigen oder proximalen nicht kreissymmetrischen Angriffsende 6.2, an dem ein Handgriff drehfest ansetzbar ist sowie einer ebenfalls im rückwärtigen oder proximalen Bereich angeordneten Graduierung 6.3 durch am Umfang quer zur Längsachse angeordneten Strichen in Form von Vertiefungen.

Die Endoahle 6 weist eine Gesamtlänge von zwischen 300 mm und 400 mm, vorzugsweise 370 mm, ein Angriffsende 6.2 zwischen 20 mm und 30 mm, vorzugsweise 25 mm, eine sich vom letzten distalen Graduierungsstrich bis zur Spitze hin erstreckende Länge zwischen 200 mm und 300 mm, vorzugsweise 250 mm sowie eine Spitzenlänge zwischen 5 mm und 15 mm, vorzugsweise 10 mm auf. Der Durchmesser einer erfindungsgemäßen Endoahle 6 liegt zwischen 2 mm und 3,5 mm, vorzugsweise im Bereich von 2,6 mm bis 3 mm. Die konische Spitze 6.1 ist in zwei Abschnitte unterteilt mit einem kürzeren Abschnitt, der eine Konizität von 6° aufweist und einem längeren Abschnitt mit einer Konizität von 17°, wobei letztere sich über etwa drei Viertel bis vier Fünftel der Gesamtlänge der konischen Spitze 6.1 erstreckt.

Die Endoahle 6 wird an innerem Material am hinteren Längsband durch axial einwirkende Kraft, wie beispielsweise mittels Hämmern verankert. Die Endoahle 6 kann dann zur Führung eines Meißelfräsers dienen, wie er weiter unten beschrieben wird.

Die Endoahle 6 wird, nachdem der Eingangsbereich in der vorstehend beschriebenen Weise erweitert wurde, endoskopisch unter Sicht, das heißt durch den Arbeitshohlraum eines Endoskops eingeführt und verankert.

Es ist möglich, dass in gewissen Fällen die Verankerung lediglich durch Vortreiben einer Endoahle 6 mit einer scharfen Spitze nicht geeignet ist oder nicht ausreicht.

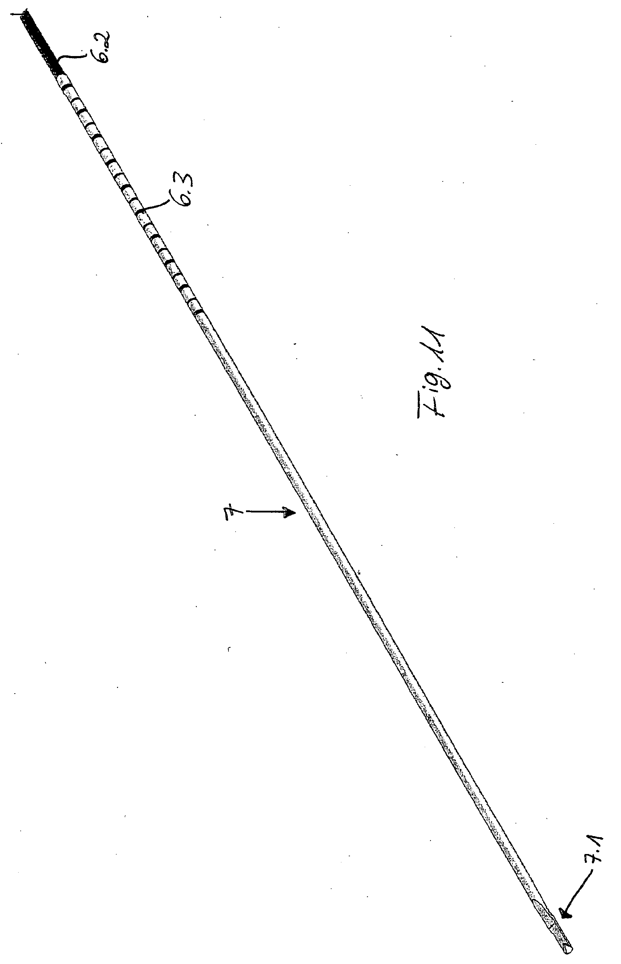

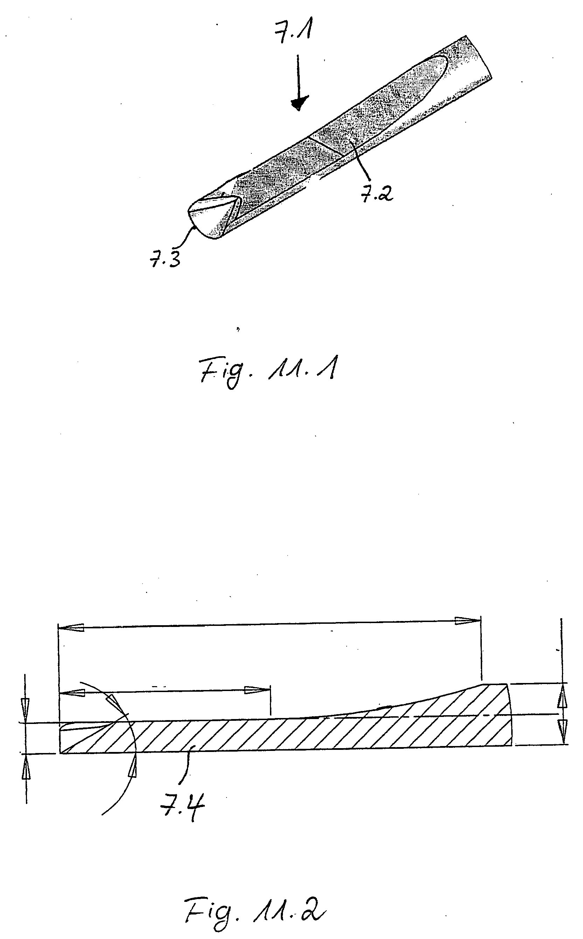

In diesem Falle sieht die Erfindung zusätzlich oder alternativ einen Endospatel 7 zur Verankerung vor, wie er in den Fig. 11 und 11.1 dargestellt ist. Auch der Endospatel 7 weist einen massiven, stabförmigen länglichen zylindrischen Körper auf. Er ist mit dem gleichen Angriffsende 6.2 sowie der gleichen Graduierung 6.3 wie die Endoahle 6 versehen, weswegen auch gleiche Bezugszeichen verwendet werden. Allerdings ist sein distaler Endbereich 7.1 deutlich anders als bei der Endoahle 6 ausgebildet. Wie insbesondere in der Fig. 11.1 erkennbar ist, weist der distale Endbereich zunächst eine bogenförmige Abflachung 7.2 auf, die dann in eine scharfe stirnseitige Kante 7.3 ausläuft, ähnlich der Kante des kanülenartigen Schneidwerkzeugs 2, wobei diese Kante allerdings auf der Außenseite 7.4 des Endospatels 7 liegt, wie insbesondere aus der Fig. 11.2 ersichtlich ist.

Der abgerundete Abflachungsbereich, ausgehend vom zylinderförmigen Hauptteil des Endospatels 7 verläuft nicht gerade schräg, sondern abgerundet mit einem Radius von vorzugsweise 35 mm. Dem schließt sich bis zum distalen Ende der Kante 7.3 des Endospatels 7 ein flacher Abschnitt an, der etwa die Stärke der Hälfte des Durchmessers des Hauptabschnitts des Endospatels 7 hat, mit einer Länge von 7 mm bis 15 mm, vorzugsweise 10 mm. Die Abkantung zur distalen scharfen Kante 7.3 hin erfolgt mit einem Winkel von etwa 25° bis 35°, vorzugsweise 30° zur Längsachse des Endospatels 7 hin.

Durch diese Ausgestaltung kann mittels der Kante des Endospatels 7, vorzugsweise unter Sicht, beispielsweise zwischen das hintere Längsband zwischen diesem und dem benachbarten Knochenbereich ein und das distale Ende des Endospatels 7 dort eingeschoben und verankert werden, um so eine bessere und zuverlässige Verankerung zu erreichen, als dies mit der Endoahle möglich ist.

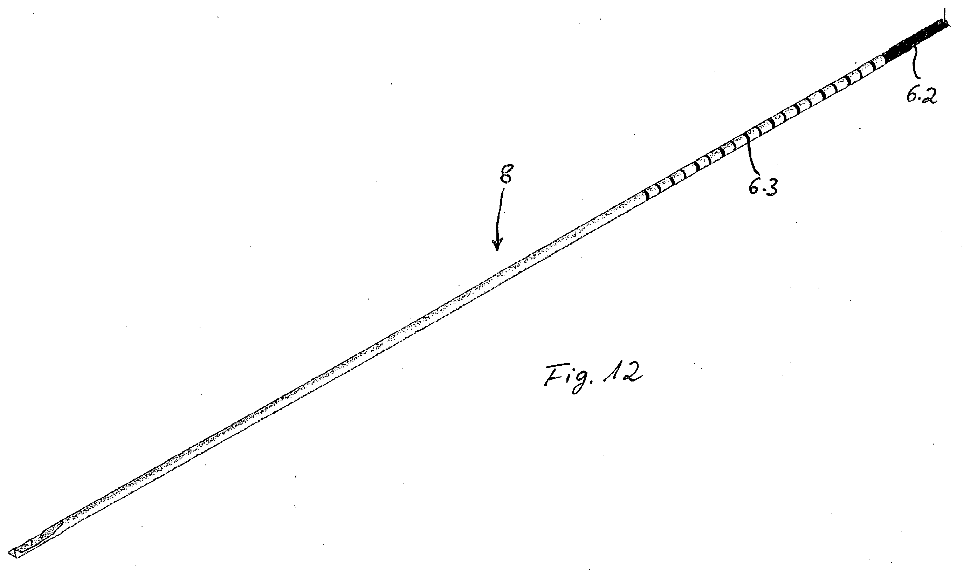

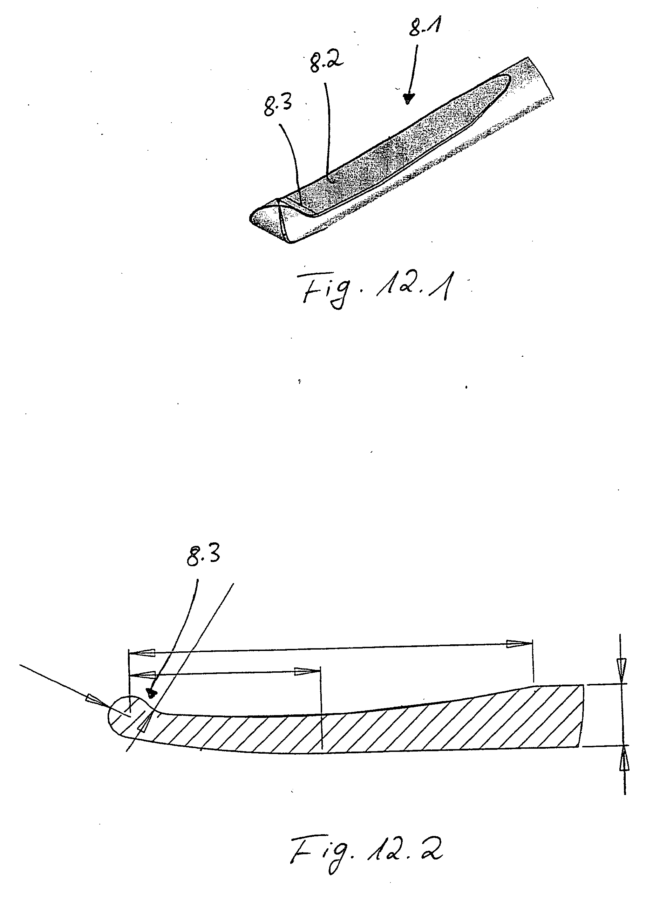

Schließlich zeigen die Fig. 12, 12.1 und 12.2 einen sogenannten Endoelevator 8, dessen distales Ende das hintere Längsband hintergreifen kann. Auch hier sind wieder gleiche Teile mit gleichen Bezugszeichen bezeichnet, so das proximale Angriffsende 6.2 und die Graduierung 6.3. Der Endoelevator 8 ist ebenfalls als massiver Hohlschaft mit einem Durchmesser in der vorstehend zur Endoahle genannten Größenordnung ausgebildet. Sein distaler Endbereich 8.1 ist ähnlich wie der des Endospatels 7 verjüngt und abgeflacht ausgebildet, bis etwa auf die Hälfte des Durchmessers des massiven Schaftes, wobei die Verjüngung über eine Verrundung mit einem Radius von 60 mm erfolgt, an den sich zunächst distal auf der verjüngten Seite hin ein flacher Bereich 8.2 anschließt, auf dessen Rückseite eine konvexe Abrundung mit einem Radius in der Größenordnung von 40 mm um eine zur Längsachse des Endoelevators senkrechte Achse gegeben ist. Das vordere Ende am Längsschnitt verdickt und teilkreisförmig ausgebildet, so dass bei 8.3 eine Hinterschneidung gegeben ist. Mit dieser kann der Endoelevator die Bänder hintergreifen und an diesen auch einen gewissen Halt auf Zug erhalten.

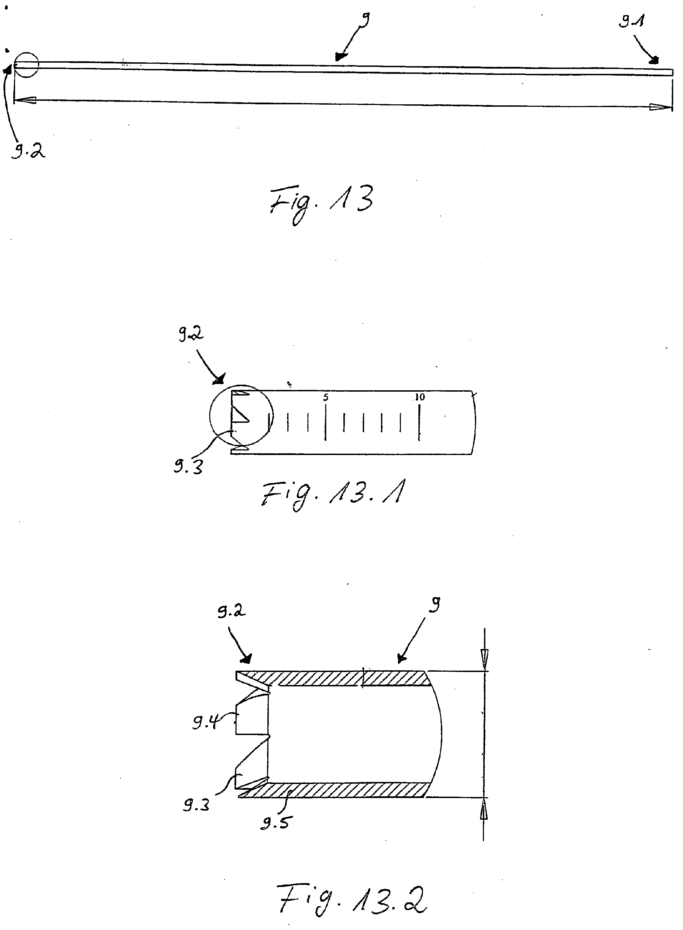

Wie schon gesagt, werden sowohl Endoahle, Endospatel als auch Endoelevator im Bereich der hinteren Längsbänder festgelegt, um als Führung für einen Meißelfräser zu dienen, wie er in den Fig. 13, 13.1 und 13.2 dargestellt ist.

Der Meißelfräser 9 weist einen gestreckten Hohlzylinder auf mit einer Länge, die etwas unterhalb der Länge von Endoahle, -Meißel, und -Elevator liegt. Das proximale Ende 9.1 (hier nicht näher ausgeführt) ist mit einer Kupplungsausgestaltung versehen, die die dreh- und axialfeste Kupplung eines Handgriffs oder Drehantriebs ermöglicht, wie eine solche Kupplung in der DE 20 2005 016 761.4 U offenbart ist, worauf verwiesen wird und die zum Offenbarungsgegenstand der vorliegenden Anmeldung gemacht wird.

Das distale Ende 9.2 ist mit einer Zähnung 9.3 versehen, wobei die Zähne zwar radial spitz zulaufen, über den Umfang hin aber eine endliche Erstreckungsrichtung, also eine Schneidkante 9.4 aufweisen. Die vordere Zahnflanke, die achsparallel gerichtet ist, weist die rückwärtige Zahnflanke zur Achse einen Winkel in der Größenordnung von 40° bis 50°, vorzugsweise 45° auf. Die Schneidkante 9.4 befindet sich auf dem Außenumfang des Mantels 9.5 des Meißelfräsers 9.

Außerdem findet sich auf der Außenseite des Mantels 9.5 des Meißelfräsers 9 im distalen Endbereich eine Graduierung, wiederum durch achssenkrechte, sich in Umfangsrichtung erstreckende Vertiefungen oder Einkerbungen, die, wenn der Meißelfräser 9 durch den Arbeitshohlraum eines Endoskops in seinen Arbeitsbereich eingeführt wird durch die seitliche Sichtoptik am distalen Ende des Endoskops gesehen und beobachtet werden kann.

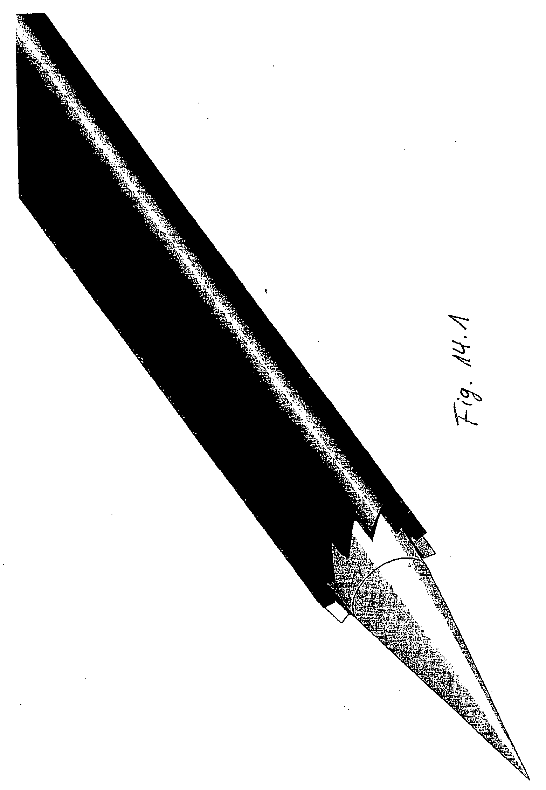

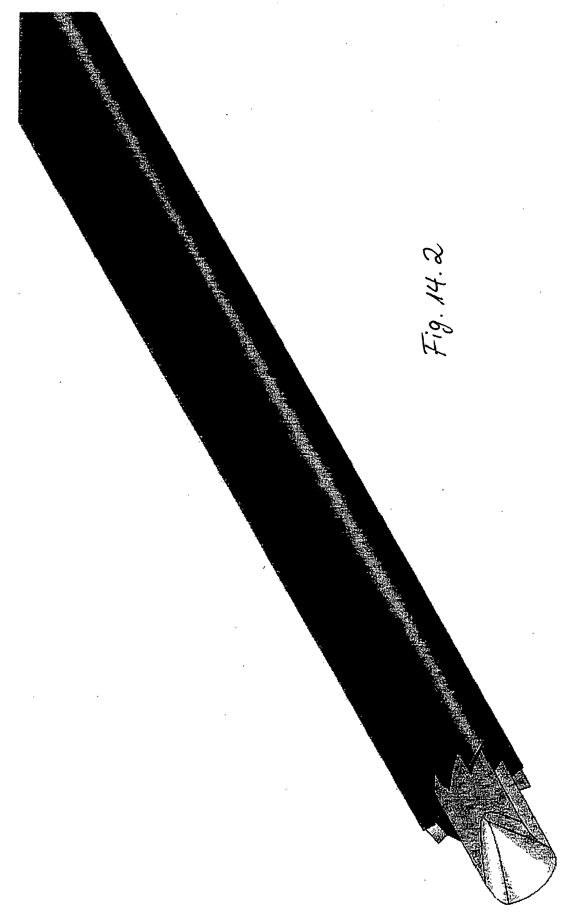

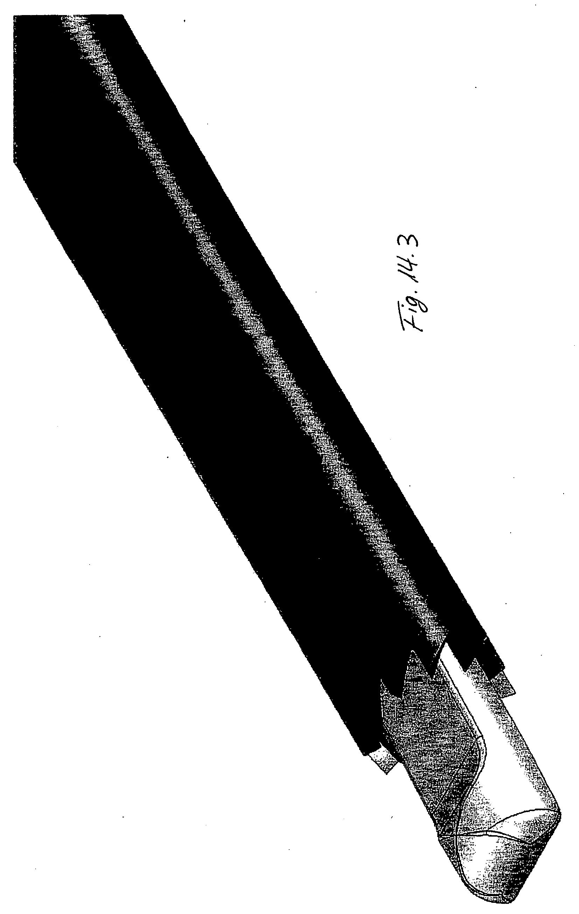

Die Fig. 14.1 bis 14.3 zeigen das Zusammenwirken eines hohlzylindrischen Meißelfräsers 9 mit einer Endoahle 6, einem Endospatel 7 bzw. einem Endoelevator 8, die sich jeweils durch den Hohlraum des Meißelfräsers hindurcherstrecken.

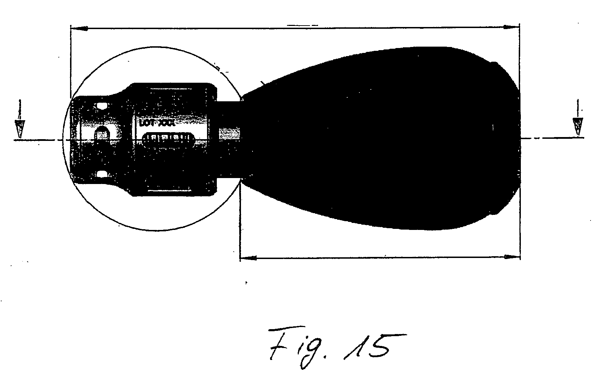

Die Fig. 15 zeigt schließlich in schematischer Darstellung einen Handgriff mit einem Antrieb in seinem Inneren sowie einer Kupplung entsprechend der DE 20 2005 016 761.4 U.

Das weitere Vorgehen unter Verwendung von Endoahle 6, -Spatel 7 oder -Elevator 8 und Meißelfräser 9 nach Einbringen des Endoskops in der oben beschriebenen Weise ist folgendes:

Durch den Arbeitskanal des sich durch das Schneidwerkzeug 2 erstreckenden Endoskops wird eines der Werkzeuge 6, 7 oder 8 bis zu dem Längsband auf der Höhe des Operationsgebiets vorbewegt und in der beschriebenen Weise dort verankert, entweder durch Einstoßen oder Einklemmen zwischen Längsband und Knochenmaterial bzw. Hinterhaken des Längsbandes.

Anschließend wird der Meißelfräser 9 über das Werkzeug 6, 7 oder 8 durch den Arbeitskanal des Endoskops eingeschoben und, wenn es an sein Arbeits- bzw. Operationsgebiet gelangt, in Drehung versetzt, so dass dort die Zähnung störendes Material, wie Knochenwucherungen oder Bänderverknorpelungen, die auf Nerven drücken, abtragen kann. Der Innendurchmesser des Meißelfräsers 9 ist dabei etwas größer als der Außendurchmesser des entsprechenden Werkzeugs 6, 7 oder 8, so dass der Meißelfräser 9 zwar durch dieses geführt wird, dennoch eine leichte Lateralbeweglichkeit möglich ist und daher eine gewisse Arbeitsfreiheit für den Operateur gegeben ist.

### Bezugszeichenliste

- 1: optische Sonde (Endoskop)
- 2: Schneidwerkzeug
- 3: Kanülen
- 4: Mittel
- 6: Endoahle
- 6.1: distales Ende
- 6.1: konische Spitze
- 6.2: Angriffsende
- 6.3: Graduierung
- 7: Endospatel
- 7.1: distaler Endbereich
- 7.2: bogenförmige Abflachung
- 7.3: Kante
- 7.4: Außenseite
- 8: Endoelevator
- 8.1: distaler Endbereich
- 8.2: flacher Bereich
- 8.3: Hinterschneidung
- 9: Meißelfräser
- 9.1: proximales Ende
- 9.2: distales Ende
- 9.3: Zähnung
- 9.4: Schneidkante
- 9.5: Mantel
- 21: Kanülenwand
- 22: distales Ende
- 23: Graduierung
- 25: Symmetrieachse
- 26: Schneide
- 26: Kante
- 27: Schnitt
- 30: Kanüle
- 101: längliches Führungselement
- 1000: Wirbelsäule
- 1001: Bandscheiben
- 1001a: Ring
- 1002: Kern
- 1003: Nervengewebe
- 1004: Wirbel
- 1005: Dornfortsatz
- 1006: Wirbelkanal
- 1007: vorderes Längsband
- 1008: hinteres Längsband
- 1009: Nerven
- 1010: Knochenwucherung
- 1011: Querfortsatz

## Patentansprüche

1. Vorrichtung zum endoskopischen Eingriff an der Wirbelsäule **gekennzeichnet durch**:
a. ein längliches Führungselement (101) mit kegelförmig abgerundeter Spitze zur Einführung einer Kanüle (2) **durch** einen Schnitt in der Haut eines Patienten,
- wobei der Innendurchmesser der Kanüle (2) dem Außendurchmesser des Führungselements (10) entspricht,
b. eine als hohles Schneidwerkzeug ausgebildete Kanüle (2) mit einem abgeschrägten distalen Ende,
- wobei der distale Bereich des distalen Endes der Kanüle (2) eine Schneide (26) aufweist, die in den Rand der Wand des Schneidwerkzeugs (2) eingearbeitet ist,
c. eine äußere hohle Kanüle (30) zur Aufnahme des Schneidwerkzeugs (2), und
d. eine optische Sonde (1) zum Einschieben **durch** den Hohlraum der Kanüle (3), wobei die optische Sonde (1) einen Arbeitskanal aufweist.

2. Vorrichtung nach Anspruch 1, **dadurch gekennzeichnet, dass** die Schneide (26) über eine kegelförmige Oberfläche mit der Außenseite der Wand des Schneidwerkzeugs (2) verbunden ist.

3. Vorrichtung nach Anspruch 1 oder 2, **gekennzeichnet durch** eine Einrichtung zum Bewegen des Schneidwerkzeugs (2), die insbesondere zum Bewegen des Schneidwerkzeugs (2) eine zyklische Bewegung in Längsrichtung des Schneidwerkzeugs (2) bewirkt und/oder eine zyklische Schwenkbewegung um eine Längs-Symmetrieachse des Schneidwerkzeugs (2), vorzugsweise um eine Längs-Symmetrieachse des Schneidwerkzeugs (2) herum über einen Schwenkradidus von jeweils bis zu 15°, insbesondere weniger als jeweils 6° in Bezug auf eine Gleichgewichtsposition bewirkt.

4. Vorrichtung nach einem der Ansprüche 1 bis 3, **dadurch gekennzeichnet, dass** die Schneide (26) sägeförmig ausgebildet ist.

5. Vorrichtung nach einem der vorangehenden Ansprüche, **dadurch gekennzeichnet, dass**
a. die optische Sonde (1) einen Arbeitskanal zum Einschieben durch den Hohlraum der Kanüle aufweist,
b. ein durch die optische Sonde (1) hindurchschiebbarer Meißelfräser (9) mit einer Zähnung (9.3) am distalen Ende (9.2) vorgesehen ist, und/oder
c. dass mindestens ein durch das Endoskop hindurchschiebbares, mit seinem distalen Ende am hinteren Längsband der Wirbelsäule festlegbares Verankerungswerkzeug (6, 7, 8) vorhanden ist.

6. Vorrichtung nach Anspruch 5, **dadurch gekennzeichnet, dass** das Verankerungswerkzeug eine Endoahle, die vorzugsweise eine scharfe distale Spitze aufweist, ein Endospatel, der vorzugsweise an seinem distalen Ende mit einer stirnseitigen Schneide versehen ist oder ein Endoelevator (8) ist, der vorzugsweise in seinem distalen Endbereich zunächst eine Verjüngung und an seinem äußersten distalen Ende wiederum eine Verdickung aufweist.

7. Vorrichtung nach einem der Ansprüche 5 oder 6, **gekennzeichnet durch** ein mit dem Verankerungswerkzeug (6, 7, 8) verbindbares Griffteil.

8. Vorrichtung nach einem der Ansprüche 5 bis 7, **gekennzeichnet durch** einen Antrieb, vorzugsweise einen Drehantrieb, insbesondere einen Drehantrieb mit Meißelwirkung für den Meißelfräser (9).

9. Vorrichtung nach einem der Ansprüche 5 bis 10, **gekennzeichnet durch** mindestens zwei der Verankerungswerkzeuge Endoahle (6), Endospatel (7) und Endoelevator (8).

10. Vorrichtung nach einem der vorangehenden Ansprüche, **dadurch gekennzeichnet, dass** die Schneide (26) am distalen Ende der Kanüle einen radialen Abstand zur Innenseite der Wand des Schneidwerkzeugs (2) aufweist, der höchstens ein Viertel des Abstandes der Schneide (26) zur Außenfläche der Wand beträgt.

11. Vorrichtung nach Anspruch 10, **dadurch gekennzeichnet, dass** die Schneide (26) sich mit dem Ende der Innenseite der Wand des Schneidwerkzeugs (2) deckt.

12. Vorrichtung nach Anspruch 10 oder 11, **dadurch gekennzeichnet, dass** der Hohlraum des Schneidwerkzeugs (2) einen Innendurchmesser zwischen 2,7 mm und 7,3 mm, vorzugsweise zwischen 3,2 mm und 6,1 mm hat.

## Claims

1. Device for endoscopic intervention on the spinal column, **characterized by**:
a. an elongate guide element (101) with a conical, rounded tip for inserting a cannula (2) through an incision in the skin of a patient,
- wherein the internal diameter of the cannula (2) corresponds to the external diameter of the guide element (10),
b. a cannula (2) designed as a hollow cutting tool with a bevelled distal end,
- wherein the distal area of the distal end of the cannula (2) has a cutting edge (26), which is incorporated into the edge of the wall of the cutting tool (2),
c. an outer hollow cannula (30) for receiving the cutting tool (2), and
d. an optical probe (1) for insertion through the cavity of the cannula (3), wherein the optical probe (1) has a working channel.

2. Device according to Claim 1, **characterized in that** the cutting edge (26) is connected to the outside of the wall of the cutting tool (2) via a conical surface.

3. Device according to Claim 1 or 2, **characterized by** a mechanism for moving the cutting tool (2), which mechanism, for the purpose of moving the cutting tool (2), in particular effects a cyclic movement in the longitudinal direction of the cutting tool (2) and/or a cyclic pivoting movement about a longitudinal axis of symmetry of the cutting tool (2), preferably about a longitudinal axis of symmetry of the cutting tool (2) over a pivot radius of in each case up to 15°, in particular of less than in each case 6°, with respect to a neutral position.

4. Device according to one of Claims 1 to 3, **characterized in that** the cutting edge (26) is serrated.

5. Device according to one of the preceding claims, **characterized in that**
a. the optical probe (1) has a working channel for insertion through the cavity of the cannula,
b. a chisel reamer (9) is provided which can be pushed through the optical probe (1) and has a toothing (9.3) at the distal end (9.2), and/or
c. at least one anchoring tool (6, 7, 8) is present which can be pushed through the endoscope and which, with its distal end, can be fixed on the posterior longitudinal ligament of the spinal column.

6. Device according to Claim 5, **characterized in that** the anchoring tool is an endo-awl, which preferably has a sharp distal tip, an endo-spatula, which is preferably provided at its distal end with a front-face cutting edge, or an endo-elevator (8) which preferably in its distal end area initially has a tapered part and then, at its outer distal end, a thickened part.

7. Device according to either of Claims 5 and 6, **characterized by** a grip part that can be connected to the anchoring tool (6, 7, 8).

8. Device according to one of Claims 5 to 7, **characterized by** a drive, preferably a rotary drive, in particular a rotary drive with chiselling action for the chisel reamer (9).

9. Device according to one of Claims 5 to 10, **characterized by** at least two of the anchoring tools endo-awl (6), endo-spatula (7) and endo-elevator (8).

10. Device according to one of the preceding claims, **characterized in that** the cutting edge (26) at the distal end of the cannula is at a radial distance from the inside of the wall of the cutting tool (2) amounting to at most a quarter of the distance between the cutting edge (26) and the outer face of the wall.

11. Device according to Claim 10, **characterized in that** the cutting edge (26) coincides with the end of the inside of the wall of the cutting tool (2).

12. Device according to Claim 10 or 11, **characterized in that** the cavity of the cutting tool (2) has an internal diameter of between 2.7 mm and 7.3 mm, preferably of between 3.2 mm and 6.1 mm.

## Revendications

1. Dispositif pour intervention endoscopique sur la colonne vertébrale, **caractérisé par** :
a. un élément de guidage (101) oblong avec une pointe en forme de cône arrondi pour introduire une canule (2) à travers une découpe dans la peau d'un patient,
- le diamètre intérieur de la canule (2) correspondant au diamètre extérieur de l'élément de guidage (10) ;
b. La canule (2) réalisée sous la forme d'un outil de coupe creux avec une extrémité distale biseautée,
- la région distale de l'extrémité distale de la canule (2) comportant une lame (26) insérée dans la bordure de la paroi de l'outil de coupe (2) ;
c. une canule (30) creuse extérieure permettant de recevoir l'outil de coupe (2) ; et
d. une sonde optique (1) à insérer à travers l'espace creux de la canule (3), la sonde optique (1) comportant un canal de travail.

2. Dispositif selon la revendication 1, **caractérisé en ce que** la lame (26) est reliée au côté extérieur de la paroi de l'outil de coupe (2) par le biais d'une surface en forme de cône.

3. Dispositif selon la revendication 1 ou 2, **caractérisé par** un dispositif de déplacement de l'outil de coupe (2), engendrant notamment un mouvement cyclique en direction longitudinale de l'outil de coupe (2) pour déplacer l'outil de coupe (2) et/ou un mouvement de pivotement cyclique autour d'un axe de symétrie longitudinal de l'outil de coupe (2), de préférence autour d'un axe de symétrie longitudinal de l'outil de coupe (2) sur un rayon de pivotement jusqu'à respectivement 15°, notamment inférieur à respectivement 6° par rapport à une position de contrepoids.

4. Dispositif selon l'une quelconque des revendications 1 à 3, **caractérisé en ce que** la lame (26) est réalisée en forme de scie.

5. Dispositif selon l'une quelconque des revendications précédentes, **caractérisé en ce que** :
a. la sonde optique (1) comporte un canal de travail à insérer à travers l'espace creux de la canule ;
b. une fraiseuse à burin (9) pouvant être poussée à travers la sonde optique (1) est prévue avec un endentement (9.3) au niveau de l'extrémité distale (9.2) ; et/ou
c. au moins un outil d'ancrage (6, 7, 8) pouvant être poussé à travers l'endoscope et pouvant être fixé, au niveau de son extrémité distale, au ligament longitudinal arrière de la colonne vertébrale est présent.

6. Dispositif selon la revendication 5, **caractérisé en ce que** l'outil d'ancrage comporte une endo-alêne comportant de préférence une pointe distale acérée, une endo-spatule de préférence pourvue au niveau de son extrémité distale d'une lame placée du côté avant ou un endo-élévateur (8) comportant de préférence dans sa région d'extrémité distale d'abord un rétrécissement puis au niveau de son extrémité distale extérieure à nouveau un épaississement.

7. Dispositif selon l'une quelconque des revendications 5 ou 6, **caractérisé par** la présence d'une partie de manche pouvant être reliée à l'outil d'ancrage (6, 7, 8).

8. Dispositif selon l'une quelconque des revendications 5 à 7, **caractérisé par** un entraînement, de préférence un entraînement pivotant, notamment un entraînement pivotant avec un effet de burin pour la fraiseuse à burin (9).

9. Dispositif selon l'une quelconque des revendications 5 à 10, **caractérisé par** la présence d'au moins deux des outils d'ancrage parmi l'endo-alêne (6), l'endo-spatule (7) et l'endo-élévateur (8).

10. Dispositif selon l'une quelconque des revendications précédentes, **caractérisé en ce que** la lame (26) comporte au niveau de l'extrémité distale de la canule un certain écartement radial par rapport au côté intérieur de la paroi de l'outil de coupe (2) s'élevant à tout au plus un quart de l'écartement de la lame (26) par rapport à la surface extérieure de la paroi.

11. Dispositif selon la revendication 10, **caractérisé en ce que** la lame (26) se recouvre avec l'extrémité du côté intérieur de la paroi de l'outil de coupe (2).

12. Dispositif selon la revendication 10 ou 11, **caractérisé en ce que** l'espace creux de l'outil de coupe (2) a un diamètre intérieur compris entre 2,7 mm et 7,3 mm, de préférence entre 3,2 mm et 6,1 mm.
